(19) Europäisches Patentamt / European Patent Office / Office européen des brevets

(11) **EP 2 400 034 B1**

(12) **EUROPEAN PATENT SPECIFICATION**

(45) Date of publication and mention
of the grant of the patent:
**24.08.2016 Bulletin 2016/34**

(51) Int Cl.:
*C12Q 1/54* *(2006.01)*　　*G01N 33/72* *(2006.01)*
*C12Q 1/00* *(2006.01)*　　*G01N 33/66* *(2006.01)*

(21) Application number: **11171086.9**

(22) Date of filing: **22.06.2011**

(54) **Diluent for preparing analytical sample**

Verdünnungsmittel zur Aufbereitung einer analytischen Probe

Diluant pour la préparation d'échantillon analytique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2010 JP 2010142245**

(43) Date of publication of application:
**28.12.2011 Bulletin 2011/52**

(73) Proprietors:
• **ARKRAY, Inc.
Minami-ku
Kyoto-shi
Kyoto 601-8045 (JP)**
• **Sekisui Medical Co., Ltd.
Tokyo 103-0027 (JP)**

(72) Inventors:
• **TAKAGI, Takeshi
Kyoto-shi
Kyoto 602-0008 (JP)**
• **SAKAI, Toshikatsu
Kyoto-shi
Kyoto 602-0008 (JP)**
• **MIYAMOTO, Chiaki
Kyoto-shi
Kyoto 602-0008 (JP)**
• **ASAMI, Takeshi
Kyoto-shi
Kyoto 602-0008 (JP)**
• **KAMATA, Tatsuo
Kyoto-shi
Kyoto 602-0008 (JP)**
• **YOTANI, Takuya
Ryugasaki-shi
Ibaraki 301-0852 (JP)**
• **OKA, Takayuki
Tokyo 103-0027 (JP)**

(74) Representative: **Golding, Louise Ann
Dehns
St Bride's House
10 Salisbury Square
London EC4Y 8JD (GB)**

(56) References cited:
**EP-A1- 2 083 274　　JP-A- 2000 346 848
US-B1- 6 670 192**

## Description

Technical Field

The present invention relates to the use of a diluent for preparing an analytical sample, which diluent is used both for analyzing a first object in a test sample by electrode method and for analyzing a second object in the test sample by liquid chromatography method.

Background Art

The method for analyzing a substance in a test sample such as a biological sample includes various methods such as electrode method and liquid chromatography method, and those methods are widely utilized in research and clinical sites.

The method for analyzing a substance by electrode method has been, for example, applied to the measurement of glucose concentration in blood by enzyme electrode method, which measurement is generally carried out in a screening test and treatment of diabetes (Japanese Laid-open Patent Application (Kokai) No. 9-33533, Japanese Laid-open Patent Application (Kokai) No. 2005-148058). According to this method, glucose in a blood sample is reacted with glucose oxidase (GOD), glucose dehydrogenase (GDH) or the like, an electric current generated by the resulting oxidation-reduction reaction of enzyme reaction product (e.g. hydrogen peroxide) or mediater such as potassium ferricyanide is measured, an operation such as equivalent point method (end point method) or differentiation (rate method) is performed to obtain the glucose concentration in the blood sample.

The method for analyzing a substance by liquid chromatography method has been, for example, applied to the measurement of glycohemoglobin concentration in blood by liquid chromatography method, which measurement is generally carried out in a screening test and treatment of diabetes (Japanese Laid-open Patent Application (Kokai) No. 5-5730, Japanese Laid-open Patent Application (Kokai) No. 9-178719). According to this method, components such as HbA1c and HbF are separated on the analytical column utilizing the difference in electric charge, and the glycohemoglobin concentration in a blood sample is obtained based on the chromatogram showing the relationship between elution times and the amounts of elution of each fraction (for example, optical information such as absorbance).

Conventionally, the above-described analysis by using electrode method and the above-described analysis by using liquid chromatography method have been carried out separately. For example, a blood analyzer for measuring glucose concentration by enzyme electrode method and a blood analyzer for measuring glycohemoglobin concentration by liquid chromatography method existed as separate apparatuses.

The present inventors sought to attain more excellent usefulness, and developed a blood analyzer which can measure both glucose concentration and glycohemoglobin concentration by using a prepared sample for measurement commonly (WO2008/035748).

Summary of Invention

In cases where a common analytical sample is used in both an analysis by electrode method and an analysis by liquid chromatography method for a test sample, there may be a problem of analytical precision since the same diluent for preparing the sample is used.

For example, in a blood analyzer in which the aforementioned measurement of glucose concentration by enzyme electrode method and the aforementioned measurement of glycohemoglobin concentration by liquid chromatography method are carried out by using the common sample for measurement, the following problem arises. Firstly, GOD electrode method as used herein utilizes fact that an electric current passes between cathode and anode by decomposing glucose at a GOD-immobilized membrane, bringing hydrogen peroxide as an enzyme reaction product to a hydrogen peroxide electrode, and applying external voltage to cause an oxidation-reduction reaction. At this time, a diluent is a supporting electrolyte solution, so that the higher the ionic strength of the diluent, the better the detection performance.

On the other hand, in liquid chromatography method as used herein, components such as HbA1c and HbF are separated by utilizing the difference in electric charge depending on the types of glycohemoglobin, so that the higher the ionic strength of the diluent, the lower the separation performance.

Therefore, it was difficult to simultaneously attain the same diluent for preparing an analytical sample which is subjected to both analysis systems of an analysis of a first object in a test sample by electrode method and an analysis of a second object in the test sample by liquid chromatography method, and high analytical precision for both two objects.

In view of these circumstances, an object of the present invention is to provide a diluent which can prepare an analytical sample used both for analyzing a first object in a test sample by electrode method and for analyzing a second object in the test sample by liquid chromatography method, and can attain high-precision measurement.

To solve the above-described problem, the present inventors intensively studied to discover that a diluent having the ionic strength range of 0.10 to 0.12 is suited for the common diluent in both analysis systems of an analysis of a first object in a test sample by electrode method and an analysis of a second object in the test sample by liquid chromatography method, thereby completing the present invention.

That is, the present invention is as follows.

(1) Use of a diluent to prepare an analytical sample, which diluent is used both for analyzing a first object in a test sample by electrode method and for analyzing a second object in the test sample by liquid chromatography method,

wherein the diluent has an ionic strength of 0.10 to 0.12;

wherein the electrode method is an electrode method using an enzyme which reacts with the first object in the test sample; and

wherein the first object is glucose and the second object is glycohemoglobin.

(2) Use according to item 1, wherein the test sample is blood.

(3) Use according to item 1 or 2, wherein said diluent contains one or more ions selected from the group consisting of sodium ion, potassium ion, calcium ion, magnesium ion, chloride ion, phosphate ion and hydrogen carbonate ion.

(4) Use according to item 2 or 3, wherein said diluent further comprises a hemolytic agent,

(5) Use according to item 4, wherein said hemolytic agent is one or more selected from the group consisting of saponin, polyoxyethylene alkyl aryl ether, higher aliphatic alcohol, alkyl aryl polyether alcohol, polyoxyethylene glycol or polyoxyethylene ether of sulfonate compound or sulfate compound, polyoxyethylene adduct of sorbitol fatty acid ester, and ammonium chloride.

(6) Use of a kit to prepare an analytical sample, which kit is used both for analyzing a first object in a blood sample by electrode method and for measuring a second object in the blood sample by liquid chromatography method, the kit comprising a diluent and a hemolytic agent;

wherein an ionic strength of the diluent after mixed with the hemolytic agent is 0.10 to 0.12;

wherein the electrode method is an electrode method using an enzyme which reacts with the first object in the sample; and

wherein the first object is glucose and the second object is glycohemoglobin.

(7) Use according to item 6, wherein said hemolytic agent is one or more selected from the group consisting of saponin, polyoxyethylene alkyl aryl ether, higher aliphatic alcohol, alkyl aryl polyether alcohol, polyoxyethylene glycol or polyoxyethylene ether of sulfonate compound or sulfate compound, polyoxyethylene adduct of sorbitol fatty acid ester, and ammonium chloride,

(8) An analytical method comprising the steps of:

mixing a diluent and a test sample to prepare the analytical sample;

a first analysis step of analyzing a first object in said analytical sample by an electrode method; and

a second analysis step of analyzing a second object in said analytical sample by a liquid chromatography method;

wherein the diluent is the diluent defined according to any one of items 1 to 5;

wherein the first object is analyzed by an electrode method using an enzyme which reacts with the first object;

wherein said first analysis step is a step of measuring glucose concentration in said analytical sample by enzyme electrode method, and

said second analysis step is a step of measuring glycohemoglobin concentration in said analytical sample by liquid chromatography method.

According to the present invention, the use of a diluent which can prepare an analytical sample used both for analyzing a first object in a test sample by electrode method and for analyzing a second object in the test sample by liquid chromatography method, and can attain high-precision measurement is provide.

BRIEF DESCRIPTION OF THE DRAWINGS

Fig. 1 is a flow chart showing the analytical method according to the present invention.
Fig. 2 is a graph showing the influence by the ionic strength of the diluent on the measurement performance of glucose concentration and hemoglobin A1c.

Mode for Carrying Out the Invention

The diluent herein described is used for preparing an analytical sample which can be used both for analyzing a first object in a test sample by electrode method and for analyzing a second object in the test sample by liquid chromatography method.

The above-described electrode method is an enzyme electrode method using an enzyme. In the above-described enzyme electrode method, as an enzyme immobilized on an electrode, the following enzymes are exemplified. Examples of such enzymes include those generally used in the enzyme electrode method, such as glucose oxidase (GOD) and glucose dehydrogenase (GDH), and preferably GOD, but are not restricted thereto.

The second object is glycohemoglobin. The measurement of glycohemoglobin by liquid chromatography method is carried out by measuring an absorbance of each fraction at the absorption wavelength of hemoglobin after separating the fraction on the analytical column. Examples of the analytical column used include those generally used in separating glycohemoglobin, which filler is silica gel, polymethacrylate, polyhydroxymethacrylate, polyvinyl alcohol, styrenedivinylbenzene copolymer or the like.

In the present invention, the test sample contains the first object and the second object, and the examples of the test sample preferably include a blood sample, but are not restricted thereto.

In the dilution for use according to the present invention, the ionic strength is 0.10 to 0.12. In the present invention, the ionic strength is one obtained by adding the products of mol concentration m of each ion and the square of charge z for all ionic species in solution, and then dividing the total into halves, and can be calculated by using the following equation (A).

$$I = (\Sigma Ci \cdot zi^2)/2 \quad (A)$$

Wherein, $Ci$ and $zi$ represent the concentration and the charge of the "i"th ion in the solution, respectively.

The test sample is diluted with the diluent as herein described until the usual dilution factor, for example, about 50 to 200 times is attained. The analytical sample prepared by using the diluent having the ionic strength within this range can be subjected to both for analyzing a first object in a test sample by electrode method and for analyzing a second object in the test sample by liquid chromatography method, and high-precision analysis can be attained. The analytical sample is especially useful for preparing a sample for measurement used both for analyzing glucose concentration in a blood sample by enzyme electrode method and for analyzing glycohemoglobin concentration in the blood sample by liquid chromatography method.

The type of an ion contained in the diluent for use according to the present invention is not restricted, for example, the ion is one or more selected from the group consisting of sodium ion ($Na^+$), potassium ion ($K^+$), calcium ion ($Ca^{2+}$), magnesium ion ($Mg^{2+}$), chloride ion ($Cl^-$), phosphate ion ($PO_4^{3-}$) and hydrogen carbonate ion ($HCO_3^-$). This is because more stable measurement system can be obtained by using the same type of ion as that of ion contained in a living body in measuring a component in the blood specimen. The valency of the ion used herein is not restricted.

The diluent for use according to the present invention is prepared by using an electrolyte which generates the above-described ion, such as an alkali metal salt or alkaline-earth metal salt.

The diluent for use according to the present invention may contain a hemolytic agent in an amount of not adversely affecting the effect of the present invention. Examples of the hemolytic agent for destroying a blood cell membrane of a blood cell component in a blood specimen include known hemolytic agents including glycoside such as saponin; surfactant such as polyoxyethylene alkyl aryl ether, higher aliphatic alcohol, alkyl aryl polyether alcohol, polyoxyethylene glycol or polyoxyethylene ether of sulfonate compound or sulfate compound, and polyoxyethylene adduct of sorbitol fatty acid ester; and ammonium chloride. The hemolytic agent may be preliminarily added to the diluent, and it may be added together with the blood specimen in preparing a sample for measurement, or may be added before or after adding the blood specimen.

Further, the diluent for use according to the present invention may contain any additive such as a known antiseptic in an appropriate amount of not adversely affecting the effect of the present invention.

For use in the present invention, the diluent can constitute part of a kit for preparing an analytical sample in combination with a hemolytic agent or a solution comprising a hemolytic agent, which kit can be used both for analyzing a first object in a blood sample by electrode method and for measuring a second object in the blood sample by liquid chromatography method. In this case, a container containing the diluent and a container containing the hemolytic agent or the solution comprising the hemolytic agent are included as separate packagings in the kit. The ionic strength when the diluent and the hemolytic agent or the solution comprising the hemolytic agent are mixed is 0.10 to 0.12.

By preparing an analytical sample with the use of this kit, the analytical sample can be subjected to both for analyzing a first object in a blood sample by electrode method and for analyzing a second object in the blood sample by liquid chromatography method.

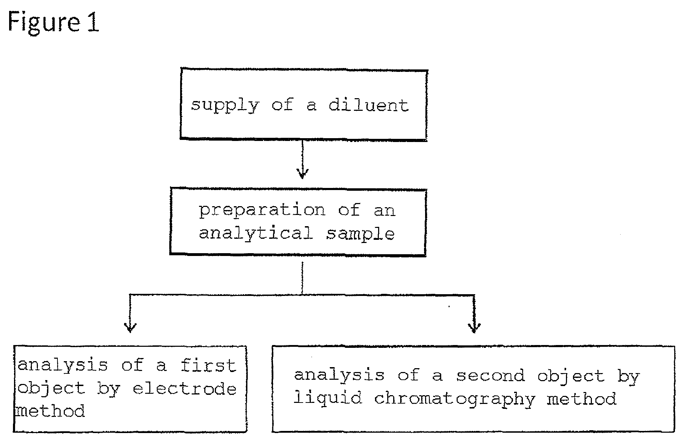

The diluent for use according to the present invention can be applied to an analytical method comprising the steps of: (i) mixing a diluent for preparing an analytical sample and a test sample to prepare the analytical sample; (ii) a first analysis step of analyzing a first object in the analytical sample by electrode method; and (iii) a second analysis step of analyzing a second object in the analytical sample by liquid chromatography method. As for the analytical method, see the flow chart shown in Fig. 1. In the analytical method, the first analysis step (ii) is a step of measuring glucose concentration in the analytical sample by enzyme electrode method, and the second analysis step (iii) is a step of measuring glycohemoglobin concentration in the analytical sample by liquid chromatography method. High-precision analysis can be attained for both the first object and the second object in the test sample by carrying out the above-mentioned analytical method with the use of the diluent as herein described as the diluent in the step (i) of preparing the analytical sample.

The diluent for use according to the present invention can be applied to analyze the first object and the second object by using an analytical apparatus having a diluent bottle, a preparation device of the analytical sample, an analytical device of the first object and an analytical device of the second object.

The diluent bottle used herein contains the diluent. In the preparation device of the sample, the diluent supplied from the diluent bottle and a test sample are mixed to prepare the analytical sample. In the analytical device of the first object, the first object is analyzed by electrode method for the analytical sample prepared in the preparation device of the sample. In the analytical device of the second object, the second object is analyzed by liquid chromatography method for the analytical sample also prepared in the preparation device of the sample. In this analytical apparatus, the diluent as herein described functions as the diluent for preparing an analytical sample, which diluent is used both for analyzing the first object in a test sample by electrode method and for analyzing the second object in the test sample by liquid chromatography method. The analytical apparatus used herein includes the apparatus wherein the analytical device of the first object is for measuring glucose concentration in the analytical sample by enzyme electrode method, and the analytical device of the second object is for measuring glycohemoglobin concentration in the analytical sample by liquid chromatography method.

The diluent for use according to the present invention also serves as a washing solution for a path and a measuring device in this analytical apparatus.

Examples

The present invention will now be described in more detail by way of Examples, but the present invention is not restricted to these Examples.

Example 1

The influence of the ionic strength of the diluent for preparing the test sample on the analytical performance of the first object and the second object was investigated. In this Example, the first object was glucose, the second object was glycohemoglobin, and each concentration of these in the test blood sample was measured by the following method to evaluate the measurement performance.

Buffers containing supporting electrolytes (alkali metal, alkaline-earth metal and the like) having different concentrations were prepared, and diluents having various ionic strengths were prepared. The glucose concentrations in the samples for measurement obtained by diluting the test blood sample with the use of these each diluent were measured by using GOD electrode. Further, the glycohemoglobin A1c concentrations in the samples for measurement were measured by liquid chromatography method using ion-exchange column. As for measuring apparatuses, the apparatuses widely used in the usual measurement were used.

As the test blood sample used herein, the sample in which both glucose concentration and glycohemoglobin A1c concentration were known was used, and the glucose concentration and the glycohemoglobin A1c concentration were 100 mg/dL and 5%, respectively. The difference between the measured value by the above-described measurement and the known concentration value was calculated as measurement accuracy (%). In cases where the ranges of the measurement accuracy are 97 to 103% for glucose concentration, and 99 to 101% for glycohemoglobin A1c concentration, it is considered that the accuracy of measurement is high. The reason why different criteria are set for each item is that glucose is greatly influenced by clinical condition of a sample donor of short time period on the order of hours to days, while glycohemoglobin A1c indicates clinical condition of a sample donor of medium- to long-term period on the order of several months and does not vary in the short time period.

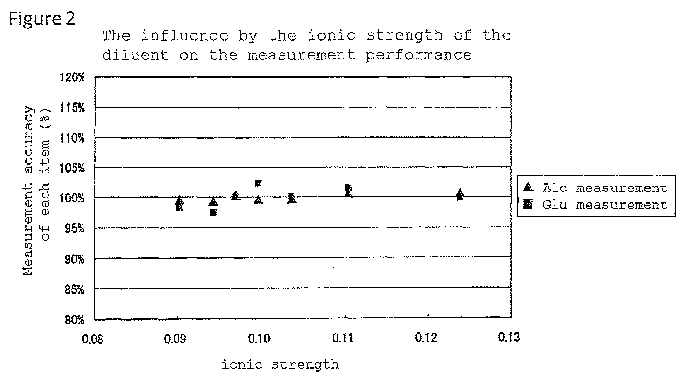

The result of each of the measurement accuracy for glucose and glycohemoglobin at each value of ionic strength of the diluent is shown in Fig. 2. By this, it can be seen that, the measurement accuracies for both glucose concentration and glycohemoglobin A1c concentration are high over the ionic strength range of the dilution from 0.090 to 0.124, and moreover, the measurement accuracies are especially high over the ionic strength range from 0.100 to 0.120.

By using the diluent as herein described, the diluent can be subjected to both for analyzing the first object in a test sample by electrode method and for analyzing the second object in the test sample by liquid chromatography method, and high-precision analysis can be attained, which is industrially useful. Especially, in cases where the diluent is applied for preparing a sample for measurement used both for measuring glucose concentration in a blood sample by enzyme electrode method and for measuring glycohemoglobin concentration in the blood sample by liquid chromatography method, the diluent is useful for a screening test and treatment of diabetes.

**Claims**

1. Use of a diluent to prepare an analytical sample, which diluent is used both for analyzing a first object in a test sample by electrode method and for analyzing a second object in the test sample by liquid chromatography method,
   wherein the diluent has an ionic strength of 0.10 to 0.12;
   wherein the electrode method is an electrode method using an enzyme which reacts with the first object in the test sample; and
   wherein the first object is glucose and the second object is glycohemoglobin.

2. Use according to claim 1, wherein the test sample is blood.

**3.** Use according to claim 1 or 2, wherein said diluent contains one or more ions selected from the group consisting of sodium ion, potassium ion, calcium ion, magnesium ion, chloride ion, phosphate ion and hydrogen carbonate ion.

**4.** Use according to claim 2 or 3, wherein said diluent further comprises a hemolytic agent.

**5.** Use according to claim 4, wherein said hemolytic agent is one or more selected from the group consisting of saponin, polyoxyethylene alkyl aryl ether, higher aliphatic alcohol, alkyl aryl polyether alcohol, polyoxyethylene glycol or polyoxyethylene ether of sulfonate compound or sulfate compound, polyoxyethylene adduct of sorbitol fatty acid ester, and ammonium chloride.

**6.** Use of a kit to prepare an analytical sample, which kit is used both for analyzing a first object in a blood sample by electrode method and for measuring a second object in the blood sample by liquid chromatography method, the kit comprising a diluent and a hemolytic agent;
wherein an ionic strength of the diluent after mixed with the hemolytic agent is 0.10 to 0.12;
wherein the electrode method is an electrode method using an enzyme which reacts with the first object in the sample; and
wherein the first object is glucose and the second object is glycohemoglobin.

**7.** Use according to claim 6, wherein said hemolytic agent is one or more selected from the group consisting of saponin, polyoxyethylene alkyl aryl ether, higher aliphatic alcohol, alkyl aryl polyether alcohol, polyoxyethylene glycol or polyoxyethylene ether of sulfonate compound or sulfate compound, polyoxyethylene adduct of sorbitol fatty acid ester, and ammonium chloride.

**8.** An analytical method comprising the steps of:

mixing a diluent and a test sample to prepare the analytical sample;
a first analysis step of analyzing a first object in said analytical sample by an electrode method; and
a second analysis step of analyzing a second object in said analytical sample by a liquid chromatography method;
wherein the diluent is the diluent defined according to any one of claims 1 to 5;
wherein the first object is analyzed by an electrode method using an enzyme which reacts with the first object;
wherein said first analysis step is a step of measuring glucose concentration in said analytical sample by enzyme electrode method, and said second analysis step is a step of measuring glycohemoglobin concentration in said analytical sample by liquid chromatography method.

**Patentansprüche**

**1.** Verwendung eines Verdünnungsmittel zum Herstellen einer Analyseprobe, welches Verdünnungsmittel sowohl zum Analysieren eines ersten Objekts in einer Testprobe durch ein Elektrodenverfahren als auch zum Analysieren eines zweiten Objekts in der Testprobe durch ein Flüssigchromatographieverfahren verwendet wird,
wobei das Verdünnungsmittel eine Ionenstärke von 0,10 bis 0,12 aufweist; wobei das Elektrodenverfahren ein Elektrodenverfahren ist, bei dem ein Enzym verwendet wird, das mit dem ersten Objekt der Testprobe reagiert; und
wobei das erste Objekt Glucose ist und das zweite Objekt Glycohämoglobin ist.

**2.** Verwendung nach Anspruch 1, wobei die Testprobe Blut ist.

**3.** Verwendung nach Anspruch 1 oder 2, wobei das Verdünnungsmittel ein oder mehrere Ionen enthält ausgewählt aus der Gruppe bestehend aus Natriumion, Kaliumion, Calciumion, Magnesiumion, Chloridion, Phosphation und Hydrogencarbonation.

**4.** Verwendung nach Anspruch 2 oder 3, wobei das Verdünnungsmittel ferner ein hämolytische Mittel umfasst.

**5.** Verwendung nach Anspruch 4, wobei das hämolytische Mittel eines oder mehrere ist ausgewählt aus der Gruppe bestehend aus Saponin, Polyoxyethylenalkylarylether, höherem aliphatischem Alkohol, Alkylarylpolyetheralkohol, Polyoxyethylenglycol oder Polyoxyethylenether einer Sulfonatverbindung oder Sulfatverbindung, einem Polyoxyethylenaddukt von Sorbitfettsäureester und Ammoniumchlorid.

**6.** Verwendung eines Kits zum Herstellen einer Analyseprobe, welches Kit sowohl zum Analysieren eines ersten Objekts in einer Blutprobe durch ein Elektrodenverfahren als auch zum Messen eines zweiten Objekts in der Blutprobe durch ein Flüssigchromatographieverfahren verwendet wird, wobei das Kit ein Verdünnungsmittel und ein hämolytisches Mittel umfasst;
wobei eine Ionenstärke des Verdünnungsmittels nach dem Mischen mit dem hämolytischen Mittel 0,10 bis 0,12 beträgt;
wobei das Elektrodenverfahren ein Elektrodenverfahren ist, bei dem ein Enzym verwendet wird, das mit dem ersten Objekt der Probe reagiert; und wobei das erste Objekt Glucose ist und das zweite Objekt Glycohämoglobin ist.

**7.** Verwendung nach Anspruch 6, wobei das hämolytische Mittel eines oder mehrere ist ausgewählt aus der Gruppe bestehend aus Saponin, Polyoxyethylenalkylarylether, höherem aliphatischem Alkohol, Alkylarylpolyetheralkohol, Polyoxyethylenglycol oder Polyoxyethylenether einer Sulfonatverbindung oder Sulfatverbindung, einem Polyoxyethylenaddukt von Sorbitfettsäureester und Ammoniumchlorid.

**8.** Analyseverfahren umfassend die Schritte von:

Mischen eines Verdünnungsmittels und einer Testprobe zum Herstellen der Analyseprobe;
einem ersten Analyseschritt des Analysierens eines ersten Objekts in der Analyseprobe durch ein Elektrodenverfahren; und
einem zweiten Analyseschritt des Analysierens eines zweiten Objekts in der Analyseprobe durch ein Flüssigchromatographieverfahren;
wobei das Verdünnungsmittel das Verdünnungsmittel ist, das einem der Ansprüche 1 bis 5 entsprechend definiert ist;
wobei das erste Objekt durch ein Elektrodenverfahren unter Anwendung eines Enzyms analysiert wird, das mit dem ersten Objekt reagiert;
wobei der erste Analyseschritt ein Schritt des Messens der Glukosekonzentration in der Analyseprobe durch ein Enzymelektrodenverfahren ist und
der zweite Analyseschritt ein Schritt des Messens der Glycohämoglobinkonzentration in der Analyseprobe durch ein Flüssigchromatographieverfahren ist.

## Revendications

**1.** Utilisation d'un diluant pour préparer un échantillon analytique, lequel diluant est utilisé à la fois pour analyser un premier objet d'un échantillon d'essai par un procédé à électrodes et pour analyser un second objet de l'échantillon d'essai par un procédé de chromatographie en phase liquide,
dans lequel le diluant a une force ionique de 0,10 à 0,12 ;
dans lequel le procédé à électrodes est un procédé à électrodes utilisant un enzyme qui réagit avec le premier objet de l'échantillon d'essai ; et
dans laquelle le premier objet est du glucose et le second objet est de la glycohémoglobine.

**2.** Utilisation selon la revendication 1, dans laquelle l'échantillon d'essai est du sang.

**3.** Utilisation selon la revendication 1 ou 2, dans laquelle ledit diluant contient un ou plusieurs ions choisis dans le groupe constitué d'un ion sodium, d'un ion potassium, d'un ion calcium, d'un ion magnésium, d'un ion chlorure, d'un ion phosphate et d'un ion hydrogénocarbonate.

**4.** Utilisation selon la revendication 2 ou 3, dans laquelle ledit diluant comprend en outre un agent hémolytique.

**5.** Utilisation selon la revendication 4, dans laquelle ledit agent hémolytique est un ou plusieurs agents choisis dans le groupe constitué de la saponine, du poly(alkylaryléther d'oxyéthylène), d'un alcool aliphatique supérieur, d'un alkylarylpolyéther alcool, d'un polyoxyéthylèneglycol ou d'un polyoxyéthylène éther de composé de sulfonate ou de composé de sulfate, d'un adduit de polyoxyéthylène d'ester d'acide gras de sorbitol et de chlorure d'ammonium.

**6.** Utilisation d'un kit pour préparer un échantillon analytique, lequel kit est utilisé à la fois pour analyser un premier objet d'un échantillon de sang par un procédé à électrodes et pour mesurer un second objet de l'échantillon de sang par un procédé de chromatographie en phase liquide, le kit comprenant un diluant et un agent hémolytique ;
dans laquelle la force ionique du diluant après mélange avec l'agent hémolytique estde0,10à0,12;
dans laquelle le procédé à électrodes est un procédé à électrodes utilisant un enzyme qui réagit avec le premier objet de l'échantillon ; et
dans laquelle le premier objet est du glucose et le second objet est de la glycohémoglobine.

**7.** Utilisation selon la revendication 6, dans laquelle ledit agent hémolytique est un ou plusieurs agents choisis dans le groupe constitué de la saponine, du poly(alkylaryléther d'oxyéthylène), d'un alcool aliphatique supérieur, d'un alkylarylpolyéther alcool, d'un polyoxyéthylèneglycol ou d'un polyoxyéthylène éther de composé de sulfonate ou de composé de sulfate, d'un adduit de polyoxyéthylène d'ester d'acide gras de sorbitol et de chlorure d'ammonium.

**8.** Procédé analytique comprenant les étapes suivantes :

le mélange d'un diluant et d'un échantillon d'essai pour préparer l'échantillon analytique ;
une première étape d'analyse visant à analyser un premier objet dudit échantillon analytique par un procédé à électrodes ; et
une seconde étape d'analyse visant à analyser un second objet dudit échantillon analytique par un procédé de chromatographie en phase liquide ;

dans lequel le diluant est le diluant défini selon l'une quelconque des revendications 1 à 5 ;

dans lequel le premier objet est analysé par un procédé à électrodes en utilisant un enzyme qui réagit avec le premier objet ;

dans lequel ladite première étape d'analyse est une étape de mesure de la concentration de glycose dans ledit échantillon analytique par un procédé enzymatique à électrodes et

ladite seconde étape d'analyse est une étape de mesure de la concentration de glycohémoglobine dans ledit échantillon analytique par un procédé de chromatographie en phase liquide.

## Figure 1

```
┌─────────────────────────────┐
│   supply of a diluent       │
└─────────────────────────────┘
                │
                ▼
┌─────────────────────────────┐
│      preparation of an      │
│      analytical sample      │
└─────────────────────────────┘
        │               │
        ▼               ▼
┌──────────────┐  ┌──────────────────────────┐
│analysis of a │  │ analysis of a second     │
│first object  │  │ object by                │
│by electrode  │  │ liquid chromatography    │
│method        │  │ method                   │
└──────────────┘  └──────────────────────────┘
```

Figure 2

The influence by the ionic strength of the
diluent on the measurement performance

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9033533 A
- JP 2005148058 A
- JP 5005730 A
- JP 9178719 A
- WO 2008035748 A